# EUROPÄISCHE PATENTSCHRIFT

(11) **EP 1 595 529 B1**
(45) Veröffentlichungstag und Bekanntmachung des Hinweises auf die Patenterteilung: **28.11.2012**
(21) Anmeldenummer: 05004261.3
(22) Anmeldetag: 26.02.2005
(51) Int. Cl.: A61K 8/02, A61Q 19/10, A61K 9/70

(54) **Kosmetische oder dermatologische getränkte Tücher**
Cosmetically or dermatologically impregnated wipes
Lingettes cosmetiques et dermatologiques imbibées

(30) Priorität: 13.05.2004 DE 102004024199
(43) Veröffentlichungstag der Anmeldung: 16.11.2005
(73) Patentinhaber: Beiersdorf AG, 20245 Hamburg (DE)
(72) Erfinder: Kohlhase, Silke, 22523 Hamburg (DE); Hahn, Ingo, 25421 Pinneberg (DE); Erkau, Herta, 22607 Hamburg (DE); Moreira, Lucinda, 22761 Hamburg (DE); Shvartzman, Michael, 15227 Kalaniot (IL); Berenstain, Yuval, 20692 Yarden (IL)

(56) Entgegenhaltungen:
- EP-A- 1 066 826
- EP-A- 1 405 632
- WO-A-03/000219
- CH-A- 784 161
- DE-A1- 4 443 639
- GB-A- 936 737
- GB-A- 2 211 092

## Beschreibung

Die vorliegende Erfindung betrifft oberflächenstrukturierte kosmetische und dermatologische Tücher, welche mit dünnflüssigen kosmetischen und dermatologischen Tränkungslösungen befeuchtet sind. Insbesondere betrifft die Erfindung kosmetische und dermatologische getränkte Pflege- und Reinigungstücher.

Getränkte Tücher finden als Gegenstände des täglichen Bedarfs breiten Einsatz in unterschiedlichsten Bereichen. Sie erlauben unter anderem effiziente und hautschonende Reinigung und Pflege besonders auch in der Abwesenheit von (fließendem) Wasser. Dabei besteht der eigentliche Gebrauchsgegenstand aus zwei Komponenten:
a) einem trockenen Tuch, welches aus Materialien wie Papier und/oder unterschiedlichsten Mischungen aus Natur- oder Kunstfasern aufgebaut ist und
b) einer niederviskosen Tränkungslösung.

Getränkte Tücher haben gegenüber kosmetischen oder dermatologischen Zubereitungen, die aus Tiegeln, Flaschen oder dergleichen entnommen und aufgebracht werden, den Vorteil, dass in ihnen die Zubereitung bereits in der richtigen Dosierung vorgegeben ist. Gegenüber einem herkömmlichen Tiegel oder einer herkömmlichen Flasche, welche -je nach verwendetem Material - leicht zerbrechen können, so dass deren Inhalt auslaufen kann, haben getränkte Tücher ferner den Vorteil, dass sie auch bei mechanischer Belastung der (flexiblen) Einsiegelung in der Regel nicht zerstört werden, so dass sie sich insbesondere für die Anwendung unterwegs oder auf Reisen eignen, nicht zuletzt auch, weil sie bequem in abgezählter Menge mitgenommen werden können.

Getränkte Tücher werden aus Textilien hergestellt. Diese Textilien können gewebt, gestrickt oder gewirkt sein oder als Verbundstoff (engl. nonwoven textile) vorliegen. Meist werden (aus Kostengründen) Verbundstoffe verwendet. Bei Verbundstoffen erfolgt die Gewebebildung nicht durch Kette und Schuss oder Maschenbildung, sondern durch Verschlingung und/oder kohäsive und/oder adhäsive Verbindung von Textilfasern. Verbundstoffe können nach der DIN 61210 T2 in Vlies, Papier Watte und Filz unterschieden werden. Vliese sind lockere Materialien aus Spinnfasern (d. h. Faser mit begrenzter Länge) oder Filamenten (Endlosfasern), meist aus Polypropylen, Polyester oder Viskose hergestellt, deren Zusammenhalt im allgemeinen durch die den Fasern eigene Haftung gegeben ist. Hierbei können die Einzelfasern eine Vorzugsrichtung aufweisen (orientierte oder Kreuzlage-Vliese) oder ungerichtet (Wirrvliese) sein. Die Vliese können mechanisch verfestigt werden durch Vernadeln, Vermaschen oder durch Verwirbeln mittels scharfer Wasserstrahlen. Adhäsiv verfestigte Vliese entstehen durch Verkleben der Fasern mit flüssigen Bindemitteln (z.B. Acrylat-Polymere, SBR/NBR, Polyvinylester, Polyurethan-Dispersionen) oder durch Schmelzen oder Auflösen von so genannten Bindefasern, die dem Vlies bei der Herstellung beigemischt wurden. Bei der kohäsiven Verfestigung werden die Faseroberflächen durch geeignete Chemikalien angelöst und durch Druck verbunden oder bei erhöhter Temperatur verschweißt [J. Falbe, M. Regnitz: Römpp-Chemie-Lexikon, 9. Aufl. Thieme-Verlag, Stuttgart (1992)].

Mit kosmetischen Zubereitungen imprägnierte Substrate und insbesondere Tücher können auf unterschiedlichen Wegen hergestellt werden: Im so genannten "Tauch-Verfahren" wird das Tuch in einem Tauchbad eingetaucht oder durch ein Bad gezogen. Dieses Verfahren eignet sich insbesondere für Papiertücher und weniger für Vliese, da letztere zu viel Flüssigkeit (= Zubereitung) aufnehmen und sich in Umverpackung anschließend Pfützen von wieder freigesetzter Zubereitung finden.

Eine zweite Variante stellt das "Sprüh-Verfahren" dar, bei dem die Zubereitung auf das vorbeilaufende Tuch aufgesprüht wird. Diese Verfahren eignet sich für alle Textilien, doch können keine stark schäumenden Zubereitungen auf das Tuch aufgebracht werden, da die Schaumentwicklung beim Sprühverfahren zu groß wird.

Als weitere Methode kommen so genannte Abstreifmethoden zum Einsatz. Dort laufen Vlies oder Tuchbahnen an Abstreifblechen, -balken oder -düsen vorbei, die kontinuierlich mit Imprägnierungslösung beladen werden. Unterschiedliche Imprägnierungsgrade lassen sich u. a. durch Variation des Anpressdruckes und der Tuchzuggeschwindigkeit einstellen.

Auch oberflächenstrukturierte Tücher sind an sich bekannt. Sie werden z. B. auf der Basis von Cellulose hergestellt und finden insbesondere als Haushaltstücher und zur perianalen Reinigung Verwendung. Ihre Struktur wird durch mechanische Prägung mittels Kalanderwalzen erzeugt. Derartige Tücher haben eine geringe Reißfestigkeit bei gleichzeitig großer Rauhigkeit und Härte. Sie eignen sich daher nur bedingt zur Verwendung an der menschlichen Haut. Ferner sind aus der DE-100 59 584-A1 auch kosmetische und dermatologische Tücher, die aus wasserstrahlverfestigten und/oder wasserstrahlgeprägten Vliesen bestehen, welche mit kosmetischen und dermatologischen Tränkungslösungen befeuchtet sind, bekannt.

Im kosmetischen oder dermatologischen Bereich eingesetzte Tücher können fernerebenso wie Bekleidungstextilien - farbig bedruckt bzw. ganz oder teilweise gefärbt sein. Dies ist insbesondere dann üblich und relativ einfach realisierbar, wenn es sich um so genannte "trockene Tücher" handelt, welche frei von Tränkungslösungen angewendet werden sollen. Derartige Tücher sind insbesondere aus dem Bereich der Toiletten- und Hygienepapiere bekannt.

Beim Färben bzw. Bedrucken wird der Farbstoff durch Adsorption an die Oberfläche, durch Eindiffundieren, durch Bildung auf und/oder in der Faser bzw. durch chemische Bindung auf das Färbegut übertragen. Zum Färben von Fasern, Garnen, Geweben, Maschenware, Vliesen und ähnlichem werden neben dem eigentlichen Farbstoff üblicherweise weitere chemische Substanzen (z. B. Färbereihilfsmittel, Salze, Alkalien, Säuren, Binder, Netzmittel, Fixierungsagentien und dergleichen) verwendet, mit deren Hilfe möglichst optimale Echtheitseigenschaften der Färbung erreicht werden sollen. Diese Stoffe können während des eigentlichen Färbevorgangs oder in einem Nachbehandlungsprozess, der sich an das eigentliche Färben anschließt, auf das Textil aufgebracht werden.

Allerdings können viele der üblicherweise im Rahmen der Färbung von Textilien verwendeten chemischen Verbindungen im Kosmetikbereich nicht eingesetzt werden. So werden z. B. im Bereich der Textilfärbung häufig Fixierungsagentien vom Melamin-Formaldehyd-Typ benutzt, welche Kondensationsprodukte aus Melamin und Formaldehyd darstellen und dementsprechend unerwünschtes freies Formaldehyd im Endprodukt freisetzen können.

Im Bereich der getränkten Kosmetik-Tücher stellt sich ferner das Problem, daß alle im Färbungsprozeß verwendeten chemischen Substanzen - insbesondere aber der eigentliche Farbstoff - kompatibel mit der eingesetzten kosmetischen oder dermatologischen Tränkungslösung sein müssen. Das bedeutet insbesondere, daß während der gesamten möglichen Lagerungsdauer des getränkten Tuches
- keine Reaktion zwischen den im Färbungsprozeß verwendeten chemischen Substanzen und den Inhaltsstoffen der Tränkungslösung erfolgen sollte und daß
- ferner die Farbe - schon aus ästhetischen Gründen - nicht aus dem Tuch herausgelöst werden darf.

Dabei stellt die Langzeitstabilität der Farbe auf dem Tuch eine besonders hohe Anforderung dar, weil getränkte kosmetische und dermatologische Tücher in feuchtem Zustand üblicherweise bis zu 30 Monate lagerbar und dementsprechend stabil sein müssen.

Aufgabe der vorliegenden Erfindung war es daher, kosmetische oder dermatologische getränkte Tücher zu finden, die die Nachteile des Standes der Technik nicht zeigen, die genannten Stabilitätsanforderungen erfüllen und sich insbesondere zur Pflege und/oder Reinigung der Haut eignen.

Es war überraschend und für den Fachmann nicht vorauszusehen, daß
kosmetische oder dermatologische Gebrauchsgegenstände, welche
A. Tücher, die Viskosefasern enthalten und die ganz oder teilweise mit Hilfe von organischen Farbstoffen aus der Gruppe der Phthalocyanine, welche für Kosmetika zugelassen sind, gefärbt und/oder bedruckt sind, wobei zum Aufbringen der Farbe auf das Tuchmaterial Polyaziridine als Fixierungsmittel verwendet werden
   und
B. kosmetische oder dermatologischen Tränkungslösungen, die - jeweils bezogen auf das Gesamtgewicht der Tränkungslösung -
   - mindestens 75 Gew.-% Wasser,
   - bis zu 15 Gew.-% Lipide und
   - bis zu 1 Gew.-% an einem oder mehreren Konservierungsmitteln enthalten, wobei der Gehalt an Phenoxyethanol kleiner oder gleich 0,6 Gew.-% gewählt wird,
umfassen
den Nachteilen des Standes der Technik abhelfen.

Zwar kennt der Fachmann die CH 7841/61, GB 2211092, WO 03/000219, EP 1066826, EP 1405632, GB 936737 und DE 4443639, doch konnten diese Schriften nicht den Weg zur vorliegenden Erfindung weisen.

Die erfindungsgemäßen Gebrauchsgegenstände stellen die Kombination eines weichen, wasserunlöslichen Tuchgewebes mit dünnflüssigen kosmetischen und dermatologischen Tränkungslösungen dar. Sie sind jeglicher Hinsicht überaus befriedigend und eignen sich dementsprechend ganz besonders, um als Grundlage für Zubereitungsformen mit vielfältigen Anwendungszwecken zu dienen. Die erfindungsgemäßen Tücher zeigen sehr gute sensorische und kosmetische Eigenschaften und zeichen sich ferner durch hervorragende Hautpflegedaten aus.

Während kosmetische oder dermatologische Tränkungslösungen, welche beispielsweise bestimmte Konservierungsmittel (wie z. B. Phenoxyethanol) oder Emulgatoren (wie z. B. Polysorbate) in höheren Konzentrationen enthalten, die Farben anlösen bzw. die getränkten Tücher sogar so stark entfärben können, daß z. B. aufgedruckte Motive kaum noch erkennbar sind, zeigen die erfindungsgemäßen Gebrauchsgegenstände keinerlei sichtbare Farbveränderung über eine Lagerungsdauer von mehr als 4 Monaten.

Die erfindungsgemäßen Tücher können glatt oder auch oberflächenstrukturiert (beispielsweise genoppt oder gelocht) sein. Erfindungsgemäß bevorzugt sind oberflächenstrukturierte Tücher.

Derartige Tücher können Makroprägungen jeden gewünschten Musters aufweisen. Die zu treffende Auswahl richtet sich zum einen nach der aufzubringenden Tränkung und zum anderen nach dem Einsatzfeld, auf dem das spätere Tuch Verwendung finden soll.

Werden geprägte Vliese verwendet, so erleichtern große Kavitäten an der Vliesoberfläche und im Vlies die Aufnahme von Schmutz und Verunreinigungen, wenn mit dem getränkten Tuch über die Haut gefahren wird. Die Reinigungswirkung kann gegenüber ungeprägten Tüchern um ein Vielfaches gesteigert werden.

Es hat sich als vorteilhaft herausgestellt für das Tuch, wenn dieses ein Gewicht von 35 bis 120 g/m², vorzugsweise von 40 bis 60 g/m², hat (gemessen bei 20 °C ± 2 °C und bei einer Feuchtigkeit der Raumluft von 65 % ± 5 % für 24 Stunden).

Die durchschnittliche Dicke des Vlieses beträgt vorzugsweise 0,4 mm bis 2 mm, insbesondere 0,6 mm bis 1,2 mm (gemessen nach der Methode ERT 30.5-99).

Als Ausgangsmaterialien für den Vliesstoff des Tuchs können neben den erfindungsgemäßen Faserstoffen generell alle organischen und anorganischen Faserstoffe auf natürlicher und synthetischer Basis verwendet werden. Beispielhaft seien Zellulose, Jute, Hanf, Sisal, Seide, Wolle, Polypropylen, Polyethylenterephthalat (PET), Aramid, Nylon, Polyvinylderivate, Polyurethane, Polylactid, Polyhydroxyalkanoat, Celluloseester und/oder Polyethylen sowie auch mineralische Fasern wie Glasfasern oder Kohlenstoffasern angeführt. Die vorliegende Erfindung ist aber nicht auf die genannten Materialien beschränkt, sondern es können eine Vielzahl weiterer Fasern zur Vliesbildung eingesetzt werden. Es ist insbesondere vorteilhaft im Sinne der vorliegenden Erfindung, wenn die eingesetzten Fasern nicht wasserlöslich sind.

Es ist erfindungsgemäß bevorzugt, wenn die Tücher ganz aus Viskosefasern bestehen.

Ferner bevorzugt im Sinne der vorliegenden Erfindung sind Tücher, welche neben den erfindungsgemäßen Viskosefasern - bezogen auf das Gesamtgewicht des Tuches - bis zu 70 Gew.-% Polyesterfasern enthalten.

Die erfindungsgemäßen Tücher können ferner vorteilhaft - jeweils bezogen auf das Gesamtgewicht des Tuches - auch bis zu 20 Gew.-% Baumwollfasern, bevorzugt - wenn sie auch Polyesterfasern aufweisen - bis zu 10 Gew.-% Baumwollfasern enthalten.

In diesem Fall ist es erfindungsgemäß insbesondere vorteilhaft, wenn das Tuch an der Oberfläche einen Baumwollanteil von bis 30 Gew.-% und im Inneren einen Baumwollanteil von bis 10 Gew.-%, jeweils bezogen auf das Gesamtgewicht des Tuchs, aufweist.

In einer weiteren bevorzugten Ausführungsform enthalten die erfindungsgemäßen Tücher 0 bis 10 Gew.-% Baumwollfasern, 40 bis 85 Gew.-% Viskosefasern und 5 bis 40 Gew.-% Polyester, jeweils bezogen auf das Gesamtgewicht des Tuches.

Darüber hinaus können die Fasern zusätzlich UV-Stabilsatoren und/oder Konservierungsmittel enthalten.

Die zur Bildung des Tuches eingesetzten Fasern weisen vorzugsweise eine Wasseraufnahmerate von mehr als >20 mm/[10 min] (gemessen mit dem EDANA Test 10.2-96), auf.

Ferner weisen die zur Bildung des Tuches eingesetzten Fasern vorzugsweise ein Wasseraufnahmevermögen von mehr als >9 g/g (gemessen mit dem EDANA Test 10.2-96), auf.

Vorteilhafte Tücher im Sinne der vorliegenden Erfindung haben eine Reißkraft von insbesondere (gemessen nach der Methode ERT 20.2-89)

| | | [N/50mm] |
|---|---|---|
| im trockenen Zustand | Maschinenrichtung | >70, vorzugsweise >80 |
| | Querrichtung | >28, vorzugsweise >30 |
| im getränkten Zustand | Maschinenrichtung | >50, vorzugsweise >60 |
| | Querrichtung | >24, vorzugsweise >30 |

Die Dehnfähigkeit vorteilhafter Tuches beträgt vorzugsweise (gemessen nach der Methode ERT 20.2-89)

| | | |
|---|---|---|
| im trockenen Zustand | Maschinenrichtung | 45 +/- 15% |
| | Querrichtung | 110 +/- 20% |
| im getränkten Zustand | Maschinenrichtung | 45 +/- 15% |
| | Querrichtung | 90 +/- 20% |

Erfindungsgemäß vorteilhaft beträgt der Reinigungsartikel der Tränkungsgrad, mit dem das Tuch mit der kosmetischen Tränkungslösung getränkt ist, von 2,1 bis 4,0 , bevorzugt von 2,4 bis 3,7 und besonders bevorzugt von 2,7 bis 3,4.

Die erfindungsgemäßen organischen Farbstoffen werden aus der Gruppe der Phthalocyanine gewählt. Ganz besonders vorteilhaft im Sinne der vorliegenden Erfindung ist das Phthalocyanin mit der Colour Index Nummer 74160.

Im Sinne der vorliegenden Erfindung werden beim Aufbringen der Farbe auf das Tuchmaterial Polyaziridine als Fixierungsmittel verwendet. Ein erfindungsgemäß besonders bevorzugtes Fixierungsmittel ist unter der Handelsbezeichnung FIXATOR ST-2 bei der AVCO CHEM erhältlich und zeichnet sich durch die folgende Strukturformel aus:

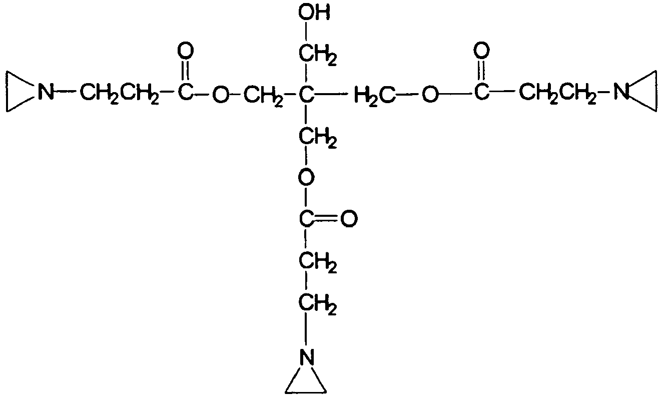

Ferner ist es bevorzugt im Sinne der vorliegenden Erfindung, wenn der Binder, der gemeinsam mit dem Fixierungsmittel die Bindung des Farbstoffs auf die Faser ermöglicht bzw. unterstützt, Carboxylgruppen enthält. Besonders bevorzugte Bindersubstanzen sind Acrylemulsionen bzw. Polyurethandispersionen. Für den Fall, dass das o. g. Fixierungsmittel FIXATOR ST-2 verwendet wird, reagieren die Carboxylgruppen mit den Aziridinringen zu einem Netzwerk und fixieren so die Farbe auf der Faser.

Es ist vorteilhaft im Sinne der vorliegenden Erfindung - insbesondere, wenn es sich um Tücher aus oberflächenstrukturiertem Vliesmaterial handelt - wenn der Farbstoff bereits während der eigentlichen Vliesherstellung (in einem "Inline-Prozess") auf das Tuchmaterial aufgebracht wird. Dies kann beispielsweise in der Art erfolgen, dass eine Wasserstrahlverfestigung, eine Wasserstrahlprägung und die Aufbringung und Fixierung der Farbe in einer Produktionslinie durchgeführt werden. Dies hat den Vorteil, dass die Tuchbahnen zum Färben nicht ein weiteres Mal ab- und aufgewickelt werden müssen, was zum einem kostspielig wäre und zum anderen die Strukturierung des Materials beeinträchtigen oder gar zerstören könnte.

Die kosmetischen und dermatologischen Tränkungslösungen, mit welchen die erfindungsgemäßen Tücher befeuchtet sind, können in verschiedenen Formen vorliegen. Sie sind vorzugsweise dünnflüssig, insbesondere sprühbar und haben z. B. eine Viskosität von weniger als 2000 mPa·s, insbesondere weniger als 1.500 mPa·s (Meßgerät: Haake Viskotester VT-02 bei 25 °C).

Die Tränkungslösungen im Sinne der vorliegenden Erfindung stellen bevorzugt Hydrodispersionen bzw. dünnflüssige Emulsionen dar. Darüberhinaus können die erfindungsgemäßen Tränkungslösungen aber auch vorteilhaft in Form von ölfreien Zubereitungen - wie beispielsweise als wäßrige bzw. wäßrig-alkoholische Lösungen - vorliegen.

Besonders vorteilhaft im Sinne der vorliegenden Erfindung sind Tränkungslösungen, welche frei von Polysorbaten sind. Das oder die Konservierungsmittel werden erfindungsgemäß vorteilhaft aus der Gruppe der Parabene, Kaliumsorbat und/oder Benzylalkohol gewählt.

Es ist ferner vorteilhaft im Sinne der vorliegenden Erfindung, wenn der pH-Wert der Tränkungslösungen zwischen 4,5 und 6,5 gewählt wird.

Die Ölphase erfindungsgemäßer Tränkungslösungen enthält - sofern sie in Form von O/W-Emulsionen vorliegen - vorteilhaft Ölkomponenten gewählt aus der Gruppe: Butylenglykoldicaprylat/-dicaprat, Dicaprylylether, C₁₂₋₁₅-Alkylbenzoat, C₁₈₋₃₈-Fettsäuretriglycerid, Dibutyladipat, Cyclomethicon.

Die Ölphase erfindungsgemäßer Tränkungslösungen enthält - sofern sie in Form von W/O-Emulsionen vorliegen - vorteilhaft Ölkomponenten gewählt aus der Gruppe: Butylenglykoldicaprylat/-dicaprat, Paraffinum Liquidum, C₁₂₋₁₅-Alkylbenzoat, C₁₈₋₃₈-Fettsäuretriglycerid, Isopropylstearat, Cetyldimethicon.

Die Ölphase der erfindungsgemäßen Tränkungslösungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianussöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung Cetiol *CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Tränkungslösungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB*) und/oder Diethylhexylnaphthalat (*Hallbrite TQ* oder *Corapan* TQ von Symrise).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 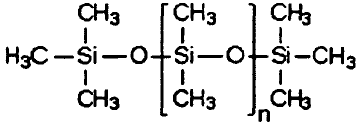 werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

### Wirkstoffe:

Besonders vorteilhafte Tränkungslösungen werden ferner erhalten, wenn als Zusatz- oder Wirkstoffe Antioxidantien eingesetzt werden. Erfindungsgemäß enthalten die Tränkungslösungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Tränkungslösungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Tränkungslösung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Tränkungslösung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Tränkungslösung, zu wählen.

Es ist insbesondere vorteilhaft, wenn die kosmetischen Tränkungslösungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin/Kreatinin (z. B. in Verhältnissen 100 : 1, bevorzugt 50 : 1 bis 3 : 1, besonders bevorzugt im Verhältnis 3 : 1), Taurin und/oder β-Alanin sowie 8-Hexadecen-1,16-dicarbonsäure (Dioic acid, CAS-Nummer 20701-68-2; vorläufige INCI-Bezeichnung Octadecendioic acid).

Weitere vorteilhaft im Sinne der vorliegenden Erfindung einzusetzende Wirkstoffe sind solche, die den Zustand der Haut positiv beeinflussen, wie insbesondere Wirkstoffe zur positiven Beeinflussung der Altershaut, die die Entstehung von Falten oder auch bestehende Falten vermindern. Vorteilhaft sind insbesondere Biochinone, insbesondere Ubichinon Q10, Carnitin, Biotin, Isoflavon, Cardiolipin, Liponsäure, Anti Freezing Proteine, Hopfen- und Hopfen-Malz-Extrakte. Auch Mittel, die die Restrukturierung des Bindegewebes fördern, wie Isoflavonoide sowie Isoflavonoid-haltige Pflanzenextrakte - wie z. B. Soja- und Klee-Extrakte - können in den erfindungsgemäßen Tränkungslösungen sehr gut verwendet werden. Auch zeigt sich, dass sich die Tränkungslösungen in besonderer Weise eignen, Wirkstoffe zur Unterstützung der Hautfunktionen bei trockener Haut (wie beispielsweise Vitamin C, Biotin, Carnitin, Propionsäure, Grüntee-Extrakte, Eucalyptusöl, Harnstoff und Mineralsalze (wie z. B. NaCl, Meeresmineralien) sowie Osmolyte (wie z. B. Inositol, Betain, quartäre Ammoniumverbindungen)) zu verwenden. In ähnlicher Weise erwies sich die Einarbeitung von Wirkstoffen zur Linderung bzw. positiven Beeinflussung von irritativen Hautzuständen, sei es bei empfindlicher Haut im allgemeinen oder bei durch Noxen gereizter Haut (UV-Licht, Chemikalien), als vorteilhaft. Hier sind Wirkstoffe zu nennen wie Sericoside, verschiedene Extrakte des Süßholzes, Licochalcone, insbesondere Licochalcon A, Silymarin, Silyphos, Dexpanthenol, Inhibitoren des Prostaglandinstoffwechsels, insbesondere der Cyclooxygenase und des Leukotrienstoffwechsels, insbesondere der 5-Lipoxyaenase, aber auch des 5-Lipoxvgenase Inhibitor Proteins, FLAP. Auch erwies sich die Einarbeitung von Modulatoren der Pigmentierung als vorteilhaft. Hier sind Wirkstoffe zu nennen, die die Pigmentierung der Haut vermindern und so zu einer kosmetisch gewünschten Aufhellung der Haut führen und/oder das Auftreten von Altersflecken reduzieren und/oder bestehende Altersflecken aufhellen. Beispielhaft sei erwähnt Tyrosinsulfat, Dioic acid (8-Hexadecen-1,16-dicarbonsäure) sowie Liponsäure und Liponamid, verschiedene Extrakte des Süßholzes, Kojisäure, Hydrochinon, Arbutin, Alpha-Arbutin, Deoxyarbutin, Fruchtsäuren, insbesondere Alpha-Hydroxy-Säuren (AHAs), Bearberry (Uvae ursi), Ursolsäure, Ascorbinsäure, Grüntee-Extrakte, Aminoguanidin, Pyridoxamin. Besonders bevorzugt sind ferner erfindungsgemäße Tränkungslösungen, die weitere Wirkstoffe enthalten, die eine verstärkte oder schnellere Bräunung der Haut herbeiführen (Advanced Glycation Endproducts (AGE), Lipofuscine, Nukleinsäure-Oligonukleotide, Purine und Pyrimidine, NO-freisetzende Substanzen), sei es mit oder ohne Einfluss von UV-Licht.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Die nachfolgenden Beispiele sollen die erfindungsgemäßen Tränkungslösungen verdeutlichen, ohne sie einzuschränken. Die Zahlenwerte in den Beispielen bedeuten Gewichtsprozente, bezogen auf das Gesamtgewicht der jeweiligen Tränkungslösungen.

### Beispiele:

| **INCI** | **1** | **2** | **3** | **4** | **5** |
|---|---|---|---|---|---|
| Sodium Hydroxide | | | | 0,02 | |
| Ceteareth-20 | 0,3 | | | | |
| Acrylates/C10-30 Alkyl Acrylate Crosspolymer | | | | 0,05 | |
| Glyceryl Stearate + Ceteareth-20 + Cetearyl Alcohol + Cetyl Palmitate + Ceteareth-12 | 1,5 | | | | |
| VP/Hexadecene Copolymer | | | | 0,5 | |
| Methylparaben | 0,3 | | 0,3 | 0,3 | 0,3 |
| Propylparaben | | | 0,07 | 0,1 | 0,07 |
| Phenoxyethanol + Methylparaben + Ethylparaben + Butylparaben + Isobutylparaben + Propylparaben | 0,5 | 0,65 | | | |
| Phenoxyethanol | | | 0,3 | 0,4 | 0,3 |
| Benzyl Alcohol | | | 0,3 | | |
| Potassium Sorbate | | 0,3 | | | 0,2 |
| Paraffinum Liquidum | 8 | | | 3 | |
| PEG-40 Hydrogenated Castor Oil | | 0,8 | 1,5 | | 1,5 |
| Glycerin | 5 | | 1 | 1 | 1 |
| Butylene Glycol | | 1 | | | |
| Ethylhexyl Stearate | 2 | | | | |
| Parfum | 0,4 | 0,2 | | 0,4 | |
| Citric Acid | | q.s. | q.s. | | q.s. |
| Sodium Citrate | | | q.s. | | q.s. |
| Aqua | ad 100 | ad 100 | ad 100 | ad 100 | ad 100 |
| Panthenol | | | 0,13 | | 0,13 |

| **INCI** | **6** | **7** | **8** | **9** | **10** | **11** | **12** |
|---|---|---|---|---|---|---|---|
| Paraffinum Liquidum | 8 | | | | | 8 | 8 |
| Isopropyl Palmitate | | 2 | 2 | | 2 | | |
| Glycerin | 5 | 5 | 5 | 5 | 5 | 5 | 5 |
| Tocopheryl Acetate | | | 0,1 | 0,1 | 0,1 | | |
| Bisabolol | | | | | | 0,1 | |
| Isohexadecane | | 3 | 3 | 3 | 3 | | |
| Ethylhexyl Stearate | 2 | | | | | 2 | 2 |
| Phenoxyethanol | 0,4 | 0,4 | 0,4 | 0,4 | 0,4 | 0,4 | 0,4 |
| Cetearyl Isononanoate | | | | 3 | | | |
| Dimethicone | | 3 | 3 | 3 | 3 | | |
| Ceteareth-20 | 0,3 | | | | | 0,3 | 0,3 |
| Panthenol | | | | | | | 0,25 |
| Sodium Hydroxide | 0 | q.s. | q.s. | q.s. | q.s. | | |
| Acrylates/C10-30 Alkyl Acrylate Crosspolymer | | 0,1 | 0,1 | | 0,1 | | |
| Carbomer | | | | 0,1 | | | |
| Glyceryl Stearate + Ceteareth-20 + Cetearyl Alcohol + Cetyl Palmitate + Ceteareth-12 | 1,5 | | | | | 1,5 | 1,5 |
| Aloe Barbadensis | 0,01 | 0,01 | | | | | |
| Polyglyceryl-3 Methylglucose Distearate | | 0,5 | 0,5 | | 0,5 | | |
| Sodium Ascorbyl Phosphate | | | 0,01 | 0,01 | 0,01 | | |
| Parfum | 0,15 | 0,3 | 0,3 | 0,3 | 0,3 | 0,3 | 0,3 |
| Maris Sal | | | | | | | 0,01 |
| Triceteareth-4 Phosphate | | | | 1 | | | |
| Butylene Glycol + lodopropynyl Butylcarbamate | | 0,1 | 0,1 | | | | |
| Methylpropanediol | | 2 | 2 | 2 | 2 | | |
| Isopropyl Isostearate | | | | 3 | 0 | | |
| Taurine | 0,1 | 0,1 | 0,1 | 0,1 | 0,1 | | |
| Methylparaben | | 0,3 | 0,3 | 0,3 | | | |
| Diazolidinyl Urea | 0,25 | | | 0,25 | 0,25 | 0,25 | 0,25 |
| Hexamidine Diisethionate | 0,05 | | | | | 0,05 | 0,05 |
| Ethylhexylglycerin | | | | 0,5 | 0,5 | | |
| Citric Acid | q.s. | | | | | | q.s. |
| Aqua | ad 100 | ad 100 | ad 100 | ad 100 | ad 100 | ad 100 | ad 100 |

## Patentansprüche

1. Kosmetische oder dermatologische Gebrauchsgegenstände, welche
A. Tücher, die Viskosefasern enthalten und die ganz oder teilweise mit Hilfe von organischen Farbstoffen aus der Gruppe der Phthalocyanine , welche für Kosmetika zugelassen sind, gefärbt und/oder bedruckt sind, wobei zum Aufbringen der Farbe auf das Tuchmaterial Polyaziridine als Fixierungsmittel verwendet werden
und
B. kosmetische oder dermatologische Tränkungslösungen, die - jeweils bezogen auf das Gesamtgewicht der Tränkungslösung -
• mindestens 75 Gew.-% Wasser,
• bis zu 15 Gew.-% Lipide und
• bis zu 1 Gew.-% an einem oder mehreren Konservierungsmitteln enthalten, wobei der Gehalt an Phenoxyethanol kleiner oder gleich 0,6 Gew.-% gewählt wird,
umfassen.

2. Gebrauchsgegenstand nach Anspruch 1, **dadurch gekennzeichnet, daß** das Tuch ganz aus Viskosefasern besteht.

3. Gebrauchsgegenstand nach Anspruch 1, **dadurch gekennzeichnet, daß** das Tuch bis zu 70 Gew.-% Polyesterfasern enthält.

4. Gebrauchsgegenstand nach einem der Ansprüche 1 oder 3, **dadurch gekennzeichnet, daß** das Tuch bis zu 20 Gew.-% Baumwollfasern enthält.

5. Gebrauchsgegenstand nach Anspruch 4, **dadurch gekennzeichnet, daß** zum Aufbringen der Farbe auf das Tuchmaterial zusätzlich ein Binder verwendet wird, der die Bindung des Farbstoffs auf die Faser ermöglicht bzw. unterstützt.

6. Gebrauchsgegenstand nach Anspruch 5, **dadurch gekennzeichnet, daß** der Binder Carboxylgruppen enthält.

7. Gebrauchsgegenstand nach Anspruch 6, **dadurch gekennzeichnet, daß** als Binder Acrylemulsionen bzw. Polyurethandispersionen verwendet werden.

## Claims

1. Cosmetic or dermatological articles of daily use which comprise
A. tissues which contain viscose fibres and which are completely or partly dyed and/or printed with the aid of organic dyes from the group consisting of the phthalocyanines, which are permitted for cosmetics, where, for applying the dye to the tissue material, polyaziridines are used as fixing agents,
and
B. cosmetic or dermatological impregnation solutions which - in each case based on the total weight of the impregnation solution - contain
• at least 75% by weight of water,
• up to 15% by weight of lipids and
• up to 1 % by weight of one or more preservatives,
where the content of phenoxyethanol is chosen to be less than or equal to 0.6% by weight.

2. Article of daily use according to Claim 1, **characterized in that** the tissue consists completely of viscose fibres.

3. Article of daily use according to Claim 1, **characterized in that** the tissue contains up to 70% by weight of polyester fibres.

4. Article of daily use according to one of Claims 1 to 3, **characterized in that** the tissue contains up to 20% by weight of cotton fibres.

5. Article of daily use according to Claim 4, **characterized in that**, for applying the dye to the tissue material, a binder is additionally used which makes possible or assists the binding of the dye to the fibres.

6. Article of daily use according to Claim 5, **characterized in that** the binder contains carboxyl groups.

7. Article of daily use according to Claim 6, **characterized in that** the binders used are acrylic emulsions or polyurethane dispersions.

## Revendications

1. Articles cosmétiques ou dermatologiques d'usage courant, qui comprennent
A. des lingettes, qui contiennent des fibres de viscose et qui sont colorées et/ou imprimées en partie ou en totalité à l'aide de colorants organiques choisis dans le groupe des phtalocyanines qui sont admises pour des cosmétiques, des polyaziridines étant utilisées comme fixateur pour l'application de la couleur sur le matériau des lingettes et
B. des solutions d'imprégnation cosmétiques ou dermatologiques qui comprennent - chaque fois par rapport au poids total de la solution d'imprégnation -
• au moins 75 % en poids d'eau,
• jusqu'à 15 % en poids de lipides et
• jusqu'à 1 % en poids d'un ou plusieurs conservateurs
la teneur en phénoxyéthanol étant choisie inférieure ou égale à 0,6 % en poids.

2. Article d'usage courant selon la revendication 1, **caractérisé en ce que** la lingette est constituée en totalité de fibres de viscose.

3. Article d'usage courant selon la revendication 1, **caractérisé en ce que** la lingette contient jusqu'à 70 % en poids de fibres de polyester.

4. Article d'usage courant selon la revendication 1 ou 3, **caractérisé en ce que** la lingette contient jusqu'à 20 % en poids de fibres de coton.

5. Article d'usage courant selon la revendication 4, **caractérisé en ce que** pour l'application de la couleur sur le matériau de la lingette on utilise en outre un liant, qui permet ou facilite la fixation du colorant sur la fibre.

6. Article d'usage courant selon la revendication 5, **caractérisé en ce que** le liant contient des groupes carboxy.

7. Article d'usage courant selon la revendication 6, **caractérisé en ce qu'**on utilise comme liant des émulsions d'acrylique ou des dispersions de polyuréthane.
